# EUROPÄISCHE PATENTSCHRIFT

(11) **EP 3 199 091 B1**
(45) Veröffentlichungstag und Bekanntmachung des Hinweises auf die Patenterteilung: **07.04.2021**
(21) Anmeldenummer: 17152095.0
(22) Anmeldetag: 19.01.2017
(51) Int. Cl.: A61B 1/00, G01M 3/26

(54) **VERFAHREN UND VORRICHTUNG ZUM AUFBEREITEN VON ENDOSKOPEN SOWIE REINIGUNGS- UND DESINFEKTIONSGERÄT FÜR ENDOSKOPE**
METHOD AND APPARATUS FOR THE TREATMENT OF ENDOSCOPES AND ENDOSCOPE WASHING AND DISINFECTING DEVICE
PROCÉDÉ ET DISPOSITIF DE TRAITEMENT D'ENDOSCOPES ET APPAREIL DE NETTOYAGE ET DE DÉSINFECTION POUR ENDOSCOPE

(30) Priorität: 28.01.2016 DE 102016201284
(43) Veröffentlichungstag der Anmeldung: 02.08.2017
(73) Patentinhaber: OLYMPUS Winter & Ibe GmbH, 22045 Hamburg (DE)
(72) Erfinder: HOPF, Oliver, 25421 Pinneberg (DE); OTTENS, Benjamin, 22309 Hamburg (DE); THATE, Henning, 22417 Hamburg (DE); WIERZOCH, Jan, 25436 Tornesch (DE)
(74) Vertreter: Seemann & Partner Patentanwälte mbB

(56) Entgegenhaltungen:
- US-A1- 2001 032 494
- US-A1- 2006 252 991
- US-A1- 2007 238 923

## Beschreibung

Die Erfindung betrifft ein Verfahren zum Aufbereiten von Endoskopen, wobei ein Endoskop an einen mit einer Druckluftquelle verbundenen Druckluftschlauch druckluftdicht angeschlossen wird und mit einer vorgegebenen oder vorgebbaren Menge oder mit einem vorgegebenen oder vorgebbaren Druck an Druckluft unter Ausbildung eines ersten Überdrucks gegenüber einem Umgebungsdruck beaufschlagt wird, wobei während einer Messperiode bei unterbrochener Verbindung zur Druckluftquelle ein Druckluftabfall gemessen wird und nach Ablauf der Messperiode oder Unterschreiten eines unteren Grenzdrucks die Druckluft ganz oder teilweise wieder abgelassen wird und aus einem Verlauf des Druckabfalls eine Dichtigkeit des Endoskops bestimmt wird, und das Endoskop anschließend unter Anwendung eines zweiten Überdrucks aufbereitet wird, der geringer ist als der erste Überdruck, sowie eine entsprechende Vorrichtung, ein Reinigungs- und Desinfektionsgerät sowie eine Verwendung.

Zur Aufbereitung von, insbesondere flexiblen, Endoskopen werden üblicherweise Reinigungs- und Desinfektionsgeräte (RDG-E) eingesetzt, die auf einem chemisch-thermischen Aufbereitungsprozess basieren. Um die Endoskope durch die Reinigungsflüssigkeit nicht zu beschädigen, ist es wichtig, dass die Hülle des Endoskops vollständig intakt ist und somit Reinigungsflüssigkeit nicht in das Innere des Endoskops eindringen kann. Dies wird im RDG-E vor dem ersten Wassereinlauf gemäß DIN EN 15883 automatisch überprüft, indem das Endoskop über eine entsprechende Schnittstelle auf einen Überdruck aufgepumpt wird. Anschließend wird der Druckabfall überwacht, wobei aus einem Druckabfall auf eine Leckage in dem Endoskop geschlossen werden kann. Bei fehlgeschlagener Prüfung bzw. zu Prozessende, also zum Ende des Reinigungs- und Desinfektionsprozesses, wird das Endoskop wieder teilweise entlüftet. Die Aufbereitung selbst erfolgt bei geringerem Überdruck im Endoskop.

Entsprechende Systeme sind aus dem RDG-E-System der Anmelderin bekannt, die als ETD-Serie verkauft wird, derzeit in der vierten Generation als ETD4. Diese Systeme umfassen einen sogenannten "Leak Tester", der den Dichtigkeitstest durchführt. Dazu wird im ETD4-System der Anmelderin ein Endoskop initial nach Programmstart zur Dichtigkeitsprüfung mit einem Überdruck von 285 mbar gegenüber dem Umgebungsdruck ("ambient pressure", AP) aufgepumpt und der Überdruck nach dem Dichtigkeitstest auf ca. 150 mbar über Umgebungsdruck reduziert. Dieser Druck wird während der Aufbereitung gehalten und kontinuierlich überwacht, auch um das Eindringen von Wasser von außen zu verhindern. Die Spülzyklen mit kaltem und warmem Wasser führen zu teils starken Temperaturänderungen, die dazu führen, dass sich der Druck im Endoskop ändert. Dies wird durch das Zugeben oder Ablassen von Druckluft in das Endoskop bzw. aus dem Endoskop heraus kompensiert.

Bei der Verwendung der Endoskope in der Endoskopie treten vereinzelt Schäden auf, bei denen Endoskope von Biopsiekanälen her punktiert werden. Es entsteht eine Mikroperforation, die bei einem gemäß DIN EN 15883 durchgeführten Test unter Umständen nicht erkannt wird, weil die Undichtigkeit eine Richtungsabhängigkeit aufweisen kann, entsprechend einem Rückschlagventil. Bei der nachfolgenden Aufbereitung wird Spül- und Klarwasser mit einem deutlich größeren Druck als 150 mbar durch die zu spülenden Kanäle geleitet. Dadurch kann dann ein Übertrag von Wasser aus der Kanalspülung in den zu schützenden Bereich des Endoskops erfolgen, was zu einer Druckerhöhung im Endoskop über 150 mbar hinaus führt. Der Leak Tester reagiert darauf mit einem Ablassen von Druckluft aus dem Endoskop, um den Überdruck von 150 mbar zu halten. Dadurch kann Wasser über den Weg der Entlüftung in die Verschlauchung eindringen und schlussendlich in den Leak Tester eingetragen werden und diesen schlimmstenfalls zerstören.

Da flexible Endoskope eine Schnittstelle aufweisen, um die Dichtigkeit der Endoskophülle zu überprüfen und die Endoskope zu entlüften, besteht die Gefahr, dass bei falscher Handhabung durch den Anwender Wasser in die eigentlich intakte Endoskophülle eindringt. Bei ausreichender Menge des Wassers, die es über die Zeit bzw. die nacheinander folgenden Reinigungszyklen akkumulieren kann, kann das Endoskop beschädigt werden. Darüber hinaus kann das Wasser über die Luft, mit der die Hülle im Rahmen des Dichtigkeitstests aufgepumpt wird, bei der anschließenden Entlüftung in das RDG-E migrieren und dieses ebenfalls beschädigen. Das Dokument US 2007/238923 A1 offenbart ein Drückintegritätsprüfgerät für Endoskope.

Demgegenüber liegt der Erfindung die Aufgabe zugrunde, insbesondere flexible, Endoskope über viele Reinigungs- und Desinfektionszyklen hinweg operativ zu halten.

Dies wird gemäß einem ersten Aspekt der Erfindung durch ein Verfahren zum Aufbereiten von Endoskopen gelöst, wobei ein Endoskop an einen mit einer Druckluftquelle verbundenen Druckluftschlauch druckluftdicht angeschlossen wird und mit einer vorgegebenen oder vorgebbaren Menge oder mit einem vorgegebenen oder vorgebbaren Druck an Druckluft unter Ausbildung eines ersten Überdrucks gegenüber einem Umgebungsdruck beaufschlagt wird, wobei während einer Messperiode bei unterbrochener Verbindung zur Druckluftquelle ein Druckluftabfall gemessen wird und nach Ablauf der Messperiode oder Unterschreiten eines unteren Grenzdrucks die Druckluft ganz oder teilweise wieder abgelassen wird und aus einem Verlauf des Druckabfalls eine Dichtigkeit des Endoskops bestimmt wird, und das Endoskop anschließend unter Anwendung eines zweiten Überdrucks aufbereitet wird, der geringer ist als der erste Überdruck, das dadurch weitergebildet ist, dass wenigstens eine erste Messung einer Feuchtigkeit der Druckluft während eines Aufpumpens des Endoskops oder während der Aufbereitung des Endoskops und eine zweite Messung einer Feuchtigkeit der Druckluft beim Ablassen der Druckluft aus dem Endoskop oder während der Aufbereitung des Endoskops erfolgt und anhand eines Unterschieds oder Trends der Feuchtigkeitsmessungen eine Feuchtigkeit im Endoskop ermittelt wird.

In den ETD-Anlagen der Anmelderin, die RDG-E-Anlagen sind, wird bereits derzeit jeweils ein Modul zur Dichtigkeitsprüfung integriert, wie oben beschrieben, welches durch eine entsprechende Verschlauchung mit dem Endoskop verbunden wird. Das Modul ist mit einem Kompressor ausgestattet, der die Hülle des Endoskops über die Verschlauchung auf einen Überdruck aufpumpt. Nach Abschluss des Dichtigkeitstests bzw. zu Prozessende des Reinigungs- und Desinfektionsvorgangs wird die Endoskophülle über denselben Weg wieder entlüftet. Dazu ist ein Entlüftungsausgang im Modul realisiert.

Das erfindungsgemäße Verfahren beruht auf dem Grundgedanken, dass zwischen dem Modul zur Dichtigkeitsprüfung und dem Endoskop ein Feuchtigkeitssensor integriert wird, der die, insbesondere absolute, Luftfeuchtigkeit während des Aufpumpvorgangs und während des Entlüftungsvorgangs misst. Befindet sich Wasser bzw. Feuchtigkeit bereits in der Endoskophülle, also im Inneren des Endoskops, so wird die Luft beim Entlüften eine höhere absolute Luftfeuchtigkeit aufweisen als beim vorangegangenen Aufpumpen. Gegebenenfalls kann auch innerhalb eines Testzyklus mit mehreren Messungen ein Trend der Luftfeuchtigkeit der Druckluft erkannt werden oder über mehrere Testzyklen bzw. Test- und Reinigungs- und Desinfektionszyklen hinweg. So kann erkannt werden, dass ein schleichender Feuchtigkeitseintrag in das Endoskop stattfindet, der aber innerhalb eines einzelnen Testvorgangs noch nicht auffällig ist.

Neben der Messung während des eigentlichen Dichtigkeitstests ist auch die Feuchtigkeitsmessung während der Aufbereitung selbst sinnvoll. Während der Aufbereitung wird ebenfalls ein Überdruck im Endoskop aufgebaut, der nach Abschluss des Aufbereitungsvorgangs abgelassen wird. So ist es möglich, zu verschiedenen Zeitpunkten sowohl zu Beginn, während und zum Abschluss der Dichtigkeitsprüfung als auch zu Beginn, während und zum Abschluss der Aufbereitung die Feuchtigkeitsmessungen durchzuführen und einen Trend zu erkennen. Da die zugeführten und abgelassenen Mengen an Druckluft bekannt sind, lassen sich auch die Messungen der Luftfeuchtigkeit der Druckluft verarbeiten und miteinander vergleichen, die einerseits bei der Dichtigkeitsprüfung und andererseits bei der Aufbereitung erfasst worden sind.

Mit dem erfindungsgemäßen Verfahren bzw. dem Vorsehen eines Feuchtigkeitssensors zur Messung der Luftfeuchtigkeit in dem Luftstrom ist eine sehr simple und unaufwändige Möglichkeit geschaffen worden, die Feuchtigkeit im Endoskop indirekt zu ermitteln, ohne eine Messung im Endoskop selbst vornehmen zu müssen. Hierzu wird eine ohnehin stattfindende Luftbeaufschlagung, die für einen anderen Zweck, nämlich die Luftdichtigkeitsprüfung, verwendet wird, als Trägermaterial für die Feuchtigkeitsmessung verwendet. Ein eigenständiger Messschritt für die Messung der Feuchte im Endoskop ist somit nicht notwendig, so dass der Test sowie die Reinigung und Desinfektion eines Endoskops nicht langwieriger wird als dies vorher bereits der Fall war.

Vorzugsweise wird bei einem Unterschied oder einem Trend der Feuchtigkeitsmessungen, der oberhalb eines vorbestimmten oder vorbestimmbaren unteren Grenzwerts liegt, eine überhöhte Feuchtigkeit im Endoskop festgestellt und ein Hinweis darauf ausgegeben. Dieser untere Grenzwert dient dazu, falsche positive Messungen auszuschließen, so dass keine unnötigen Trocknungsvorgänge eingeleitet werden. Der untere Grenzwert kann endoskoptypspezifisch eingestellt werden oder in Abhängigkeit von Parametern der Dichtigkeitsmessung, wie beispielsweise dem erreichten Überdruck oder der Luftmenge, wobei physikalische Gegebenheiten zu berücksichtigen sind wie die Temperatur und der Luftdruck der Überdruckatmosphäre und die Aufnahmekapazität der Überdruckluft für Feuchtigkeit in Abhängigkeit von diesen Parametern. Diese Parameter und Abhängigkeiten sind physikalisch bekannt. Alternativ kann der untere Grenzwert auch rein empirisch ermittelt und eingestellt werden.

Vorteilhafterweise wird bei Vorliegen einer überhöhten Feuchtigkeit im Endoskop vor oder nach einer Reinigung und Desinfizierung des Endoskops eine zusätzliche Trocknung eines Innenraums des Endoskops und/oder eines Kanalsystems eines Reinigungs- und Desinfektionsgeräts und/oder einer Vorrichtung zur Dichtigkeitsüberprüfung durchgeführt.

In einer vorteilhaften Weiterbildung ist vorgesehen, dass bei einer stark überhöhten Feuchtigkeit im Endoskop oberhalb eines zweiten Grenzwerts, der oberhalb des vorbestimmten oder vorbestimmbaren unteren Grenzwerts liegt, oder bei mehrmaligem, insbesondere wenigstens zweimaligen, Auftreten einer überhöhten Feuchtigkeit im Endoskop eine Beschädigung des Endoskops festgestellt und ein Hinweis darauf ausgegeben wird. Die Begriffe "überhöhte Feuchtigkeit" und "stark überhöhte Feuchtigkeit" sind im Zusammenhang mit der vorliegenden Erfindung als Überschreiten von entsprechenden Grenzwerten zu verstehen. Der zweite Grenzwert für das Auftreten einer stark überhöhten Feuchtigkeit im Endoskop wird zweckmäßigerweise, aber nicht ausschließlich, dahingehend festgelegt, dass Erfahrungswerte verwendet werden, wonach bei entsprechend hohen Feuchtigkeitswerten tatsächliche Beschädigungen in typgleichen oder ähnlichen Endoskopen festgestellt worden sind. Ein zweiter Grenzwert für eine stark überhöhte Feuchtigkeit kann aber auch etwas niedriger eingestellt werden, um eine Warnung vor tatsächlich möglichen Beschädigungen bereitzustellen, bevor diese eintreten.

Die der Erfindung zugrunde liegende Aufgabe wird auch durch eine Vorrichtung zum Aufbereiten von Endoskopen gelöst, umfassend eine Druckluftquelle, einen Druckluftschlauch, eine Luftdruckmessvorrichtung, eine Spüleinheit und eine Steuervorrichtung, wobei der Druckluftschlauch einerseits mit der Druckluftquelle und andererseits mit einem aufzubereitenden Endoskop verbunden oder verbindbar ist, wobei die Luftdruckmessvorrichtung ausgebildet ist, einen Luftdruck eines zu einem mit der Druckluftquelle über den Druckluftschlauch verbundenen Endoskops zu messen, wobei die Steuervorrichtung ausgebildet und eingerichtet ist, für eine Dichtigkeitsprüfung das Endoskop mit Druckluft aus der Druckluftquelle auf einen ersten Überdruck gegenüber einem Umgebungsdruck zu beaufschlagen, bei unterbrochener Verbindung zur Druckluftquelle einen zeitlich abhängigen Druckluftabfall bei dem mit Druckluft beaufschlagten Endoskop zu messen, nach Ablauf der Messperiode oder Unterschreiten eines unteren Grenzdrucks die Druckluft ganz oder teilweise wieder abzulassen und aus einem Verlauf des Druckabfalls eine Dichtigkeit des Endoskops zu bestimmen, und das Endoskop anschließend unter Anwendung eines zweiten Überdrucks aufzubereiten, der geringer ist als der erste Überdruck, die dadurch weitergebildet ist, dass zusätzlich ein Feuchtigkeitssensor umfasst ist, der über eine Signal- und/oder Datenverbindung mit der Steuervorrichtung verbunden ist und ausgebildet und angeordnet ist, eine Feuchtigkeit in einem Luftvolumen zwischen dem Endoskop und der Druckluftquelle, insbesondere im Druckluftschlauch, zu messen.

Auch die Vorrichtung weist einen Feuchtigkeitssensor zusätzlich auf, der dazu ausgebildet und angeordnet ist, eine Feuchtigkeit in einem Luftvolumen zwischen dem Endoskop und der Druckluftquelle zu messen, also an einer Stelle, die beim Aufpumpen oder Ablassen von Druckluft von dem Druckluftstrom durchströmt wird, so dass Feuchtigkeit im Druckluftstrom gemessen wird. Damit vereint die erfindungsgemäße Vorrichtung die Merkmale, Vorteile und Eigenschaften des erfindungsgemäßen Verfahrens in sich.

Vorteilhafterweise ist die Steuervorrichtung ausgebildet und eingerichtet, ein erfindungsgemäßes zuvor beschriebenes Verfahren durchzuführen und aus einem Unterschied oder Trend in Feuchtigkeitsmessungen der Druckluft beim Aufpumpen des Endoskops und Ablassen der Druckluft aus dem Endoskop eine Feuchtigkeit im Endoskop zu ermitteln.

Die der Erfindung zugrunde liegende Aufgabe wird auch durch ein Reinigungs- und Desinfektionsgerät für Endoskope mit einer zuvor beschriebenen erfindungsgemäßen Vorrichtung zur Überprüfung der Dichtigkeit von Endoskopen gelöst. Auch das Reinigungs- und Desinfektionsgerät weist daher die entsprechenden Vorteile, Eigenschaften und Merkmale auf, die zu dem Verfahren und zu der Vorrichtung beschrieben worden waren.

In einer vorteilhaften Weiterbildung umfasst die Steuereinheit des Reinigungs- und Desinfektionsgeräts die Steuervorrichtung der erfindungsgemäßen Vorrichtung zur Überprüfung der Dichtigkeit von Endoskopen und ist ausgebildet und eingerichtet, nach Feststellung einer überhöhten Feuchtigkeit in einem Endoskop einen Trocknungsvorgang für das Innere des Endoskops und/oder eines Kanalsystems eines Reinigungs- und Desinfektionsgeräts und/oder einer Vorrichtung zur Dichtigkeitsüberprüfung vor oder nach einem Reinigungs- und/oder Desinfektionsvorgang einzuschalten.

Die der Erfindung zugrunde liegende Aufgabe wird auch durch eine Verwendung eines Luftfeuchtigkeitssensors in einer Vorrichtung zur Dichtigkeitsüberprüfung eines Reinigungs- und Desinfektionsgeräts für Endoskope zur Feststellung einer Feuchtigkeit in einem Endoskop gelöst. Diese Verwendung verwirklicht die oben beschriebenen erfindungsgemäßen Merkmale, Vorteile und Eigenschaften.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Darstellung eines erfindungsgemäßen Reinigungs- und Desinfektionsgerät und
- Fig. 2: eine schematische Darstellung eines erfindungsgemäßen Verfahrens.

In den Zeichnungen sind jeweils gleiche oder gleichartige Elemente und/oder Teile mit denselben Bezugsziffern versehen, so dass von einer erneuten Vorstellung jeweils abgesehen wird.

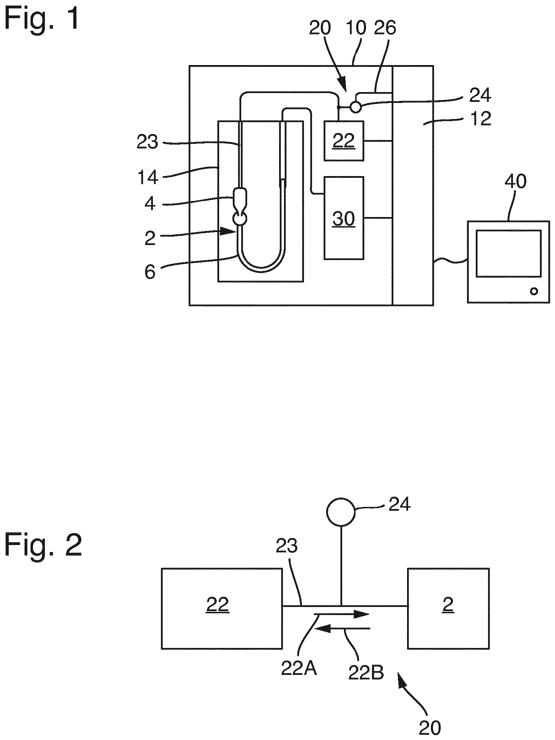

Fig. 1 zeigt eine schematische Darstellung eines erfindungsgemäßen Reinigungs- und Desinfektionsgeräts 10, in dessen Reinigungskammer 14 ein flexibles Endoskop 2 angeordnet ist, das bereit ist, gereinigt und desinfiziert zu werden. Das Endoskop 2 umfasst einen Griff 4 sowie einen flexiblen Schaft 6, die beide an Anschlüsse des Reinigungs- und Desinfektionsgeräts 10 angeschlossen sind.

Der Schaft 6 des Endoskops 2 ist über einen Anschluss mit einer Spüleinheit 30 des Reinigungs- und Desinfektionsgeräts 10 verbunden, während der Handgriff 4 über einen Druckluftschlauch 23 mit einer Druckluftquelle 22, beispielsweise einem Kompressor, mit der Vorrichtung 20 zur Dichtigkeitsüberprüfung verbunden ist. Diese ist, ebenso wie die Spüleinheit 30, mit einer Steuervorrichtung 12 des Reinigungs- und Desinfektionsgeräts 10 verbunden, die somit auch eine Steuervorrichtung für die Vorrichtung 20 zur Dichtigkeitsprüfung darstellt.

Ausgangs der Druckluftquelle 22 ist ein Feuchtigkeitssensor 24 an den Druckluftschlauch 23 angeschlossen, der die Feuchtigkeit der Luft in dem Druckluftschlauch 23 misst und die Signale über eine Signalleitung 26 ebenfalls an die Steuervorrichtung 12 des Reinigungs- und Desinfektionsgeräts 10 übermittelt. Die Steuervorrichtung 12 ist außerdem mit einer Anzeigevorrichtung 40 verbunden, mittels der die Steuervorrichtung 12 von außen beeinflusst werden kann und umgekehrt Daten aus der Steuervorrichtung 12, beispielsweise Feuchtigkeitsmessdaten von dem Feuchtigkeitssensor 24 oder Prozessdaten, angezeigt werden können.

Fig. 2 zeigt schematisch einen Teil der Vorrichtung 20 zur Dichtigkeitsüberprüfung, anhand dessen das erfindungsgemäße Verfahren erläutert wird. Dargestellt sind die Druckluftquelle 22 der Vorrichtung 20, die über einen Druckluftschlauch 23 mit einem nur schematisch angedeuteten Endoskop 2 verbunden ist und Druckluft in das Endoskop 2 pumpen kann und es auch wieder entlüften kann. Entsprechend ist mit jeweils einem Pfeil ein Volumenstrom 22A von Druckluft beim Aufpumpen und ein Volumenstrom 22B beim Ablassen von Druckluft gezeigt, wobei der Volumenstrom 22A von der Druckluftquelle 22 zum Endoskop 2 hin verläuft, während der Volumenstrom 22B beim Ablassen von Druckluft vom Endoskop 2 in Richtung auf die Druckluftquelle 22 verläuft, die einen nicht dargestellten Auslass zum Auslassen der Druckluft aufweist.

Mit dem Druckluftschlauch 23 ist ein Feuchtigkeitssensor 24 verbunden, der die Feuchtigkeit des Volumenstroms an Druckluft im Druckluftschlauch 23 misst. Dies erfolgt wahlweise fortlaufend oder zum Zeitpunkt des Aufpumpens des Endoskops 2 und des Ablassens von Druckluft aus dem Endoskop 2. Maßgeblich ist die Feuchtigkeit, die der Volumenstrom einerseits beim Aufpumpen aufweist und andererseits beim Ablassen aus dem Endoskop 2. Die Luft aus dem Volumenstrom, der vom Kompressor 22 in das Endoskop 2 strömt, weist eine Basisfeuchtigkeit auf, die noch nicht von gegebenenfalls im Endoskop 2 vorhandener Feuchtigkeit beeinflusst ist und somit eine Referenzgröße darstellt. Der Kompressor 22 weist gegebenenfalls eine Lufttrocknungseinheit auf, mittels der Feuchtigkeit aus der Luft bzw. der Atmosphäre entfernt wird, die nachfolgend komprimiert und zum Endoskop 2 geführt wird. Der Volumenstrom 22B umfasst Luft, die sich eine Weile im Endoskop 2 aufgehalten hat und somit Feuchtigkeit aus dem Endoskop 2 aufgenommen hat. Die Differenz der Feuchtigkeit des Volumenstroms 22B zum Volumenstrom 22A ist somit ein Maß für die Menge an Feuchtigkeit, die im Endoskop 2 enthalten ist. Die Menge an Luft, die als Druckluft in das Endoskop 2 gepumpt wird, ist an sich um ein Vielfaches zu klein, um einen Trocknungsprozess zu bewirken, so dass die Messung ein im Wesentlichen unverfälschtes Maß für die Feuchtigkeit im Endoskop darstellt.

Anhand des gemessenen Feuchtigkeitsunterschieds und der daraus berechneten Menge an Feuchtigkeit im Endoskop kann entschieden werden, ob ein Trocknungsvorgang für das Innere des Endoskops 2 durchgeführt werden muss, oder ob die Feuchtigkeit bereits ein Maß erreicht hat, das Beschädigungen im Endoskop befürchten lässt. Es sind dann entsprechende Maßnahmen zu ergreifen, das Endoskop entweder außer Betrieb zu nehmen oder es zu warten und gegebenenfalls zu reparieren. Dadurch wird auch die Testeinrichtung vor Überfeuchtung geschützt.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: Endoskop
- 4: Griff
- 6: Schaft
- 10: Reinigungs- und Desinfektionsgerät
- 12: Steuervorrichtung
- 14: Reinigungskammer
- 20: Vorrichtung zur Dichtigkeitsüberprüfung
- 22: Druckluftquelle
- 22A: Volumenstrom beim Aufpumpen
- 22B: Volumenstrom beim Ablassen von Druckluft
- 23: Druckluftschlauch
- 24: Feuchtigkeitssensor
- 26: Signalleitung
- 30: Spüleinheit
- 40: Anzeigevorrichtung

## Patentansprüche

1. Verfahren zum Aufbereiten von Endoskopen, wobei ein Endoskop (2) an einen mit einer Druckluftquelle (22) verbundenen Druckluftschlauch (23) druckluftdicht angeschlossen wird und mit einer vorgegebenen oder vorgebbaren Menge oder mit einem vorgegebenen oder vorgebbaren Druck an Druckluft unter Ausbildung eines ersten Überdrucks gegenüber einem Umgebungsdruck beaufschlagt wird, wobei während einer Messperiode bei unterbrochener Verbindung zur Druckluftquelle (22) ein Druckluftabfall gemessen wird und nach Ablauf der Messperiode oder Unterschreiten eines unteren Grenzdrucks die Druckluft ganz oder teilweise wieder abgelassen wird und aus einem Verlauf des Druckabfalls eine Dichtigkeit des Endoskops (2) bestimmt wird, und das Endoskop (2) anschließend unter Anwendung eines zweiten Überdrucks aufbereitet wird, der geringer ist als der erste Überdruck, **dadurch gekennzeichnet, dass** wenigstens eine erste Messung einer Feuchtigkeit der Druckluft während eines Aufpumpens des Endoskops (2) oder während der Aufbereitung des Endoskops (2) und wenigstens eine zweite Messung einer Feuchtigkeit der Druckluft zu einem späteren Zeitpunkt beim Ablassen der Druckluft aus dem Endoskop (2) oder während der Aufbereitung des Endoskops (2) erfolgt und anhand eines Unterschieds oder Trends der Feuchtigkeitsmessungen eine Feuchtigkeit im Endoskop ermittelt wird.

2. Verfahren nach Anspruch 1, **dadurch gekennzeichnet, dass** bei einem Unterschied oder einem Trend der Feuchtigkeitsmessungen, der oberhalb eines vorbestimmten oder vorbestimmbaren unteren Grenzwerts liegt, eine überhöhte Feuchtigkeit im Endoskop (2) festgestellt und ein Hinweis darauf ausgegeben wird.

3. Verfahren nach Anspruch 1 oder 2, **dadurch gekennzeichnet, dass** bei Vorliegen einer überhöhten Feuchtigkeit im Endoskop (2) vor oder nach einer Reinigung und Desinfizierung des Endoskops (2) eine zusätzliche Trocknung eines Innenraums des Endoskops (2) und/oder eines Kanalsystems eines Reinigungs- und Desinfektionsgeräts (10) und/oder einer Vorrichtung zur Dichtigkeitsüberprüfung (20) durchgeführt wird.

4. Verfahren nach einem der Ansprüche 1 bis 3, **dadurch gekennzeichnet, dass** bei einer stark überhöhten Feuchtigkeit im Endoskop (2) oberhalb eines zweiten Grenzwerts, der oberhalb des vorbestimmten oder vorbestimmbaren unteren Grenzwerts liegt, oder bei mehrmaligem, insbesondere wenigstens zweimaligem, Auftreten einer überhöhten Feuchtigkeit im Endoskop (2) eine Beschädigung des Endoskops (2) festgestellt und ein Hinweis darauf ausgegeben wird.

5. Vorrichtung zum Aufbereiten von Endoskopen, umfassend eine Druckluftquelle (22), einen Druckluftschlauch (23), eine Luftdruckmessvorrichtung, eine Spüleinheit (30) und eine Steuervorrichtung (12), wobei der Druckluftschlauch einerseits mit der Druckluftquelle (23) und andererseits mit einem aufzubereitenden Endoskop (2) verbunden oder verbindbar ist, wobei die Luftdruckmessvorrichtung ausgebildet ist, einen Luftdruck einer zu einem mit der Druckluftquelle (22) über den Druckluftschlauch (23) verbundenen Endoskop (2) zu messen, wobei die Steuervorrichtung (12) ausgebildet und eingerichtet ist, für eine Dichtigkeitsprüfung das Endoskop (2) mit Druckluft aus der Druckluftquelle (22) auf einen ersten Überdruck gegenüber einem Umgebungsdruck zu beaufschlagen, bei unterbrochener Verbindung zur Druckluftquelle (22) einen zeitlich abhängigen Druckluftabfall bei dem mit Druckluft beaufschlagten Endoskop (2) zu messen, nach Ablauf der Messperiode oder Unterschreiten eines unteren Grenzdrucks die Druckluft ganz oder teilweise wieder abzulassen und aus einem Verlauf des Druckabfalls eine Dichtigkeit des Endoskops (2) zu bestimmen, und das Endoskop (2) anschließend unter Anwendung eines zweiten Überdrucks aufzubereiten, der geringer ist als der erste Überdruck, **dadurch gekennzeichnet, dass** zusätzlich ein Feuchtigkeitssensor (24) umfasst ist, der über eine Signal- und/oder Datenverbindung (26) mit der Steuervorrichtung (12) verbunden ist und ausgebildet und angeordnet ist, eine Feuchtigkeit in einem Luftvolumen zwischen dem Endoskop (2) und der Druckluftquelle (22), insbesondere im Druckluftschlauch (23), zu messen.

6. Vorrichtung (20) nach Anspruch 5, **dadurch gekennzeichnet, dass** die Steuervorrichtung (12) ausgebildet und eingerichtet ist, ein Verfahren nach einem der Ansprüche 1 bis 5 durchzuführen und aus einem Unterschied oder Trend in Feuchtigkeitsmessungen der Druckluft beim Aufpumpen des Endoskops (2) und Ablassen der Druckluft aus dem Endoskop (2) eine Feuchtigkeit im Endoskop (2) zu ermitteln.

7. Reinigungs- und Desinfektionsgerät (10) für Endoskope mit einer Vorrichtung (20) zur Überprüfung der Dichtigkeit von Endoskopen nach Anspruch 5 oder 6.

8. Reinigungs- und Desinfektionsgerät (10) für Endoskope nach Anspruch 7, **dadurch gekennzeichnet, dass** eine Steuereinheit des Reinigungs- und Desinfektionsgeräts (10) die Steuervorrichtung (12) der Vorrichtung (20) zur Überprüfung der Dichtigkeit von Endoskopen umfasst und ausgebildet und eingerichtet ist, nach Feststellung einer überhöhten Feuchtigkeit in einem Endoskop (2) einen Trocknungsvorgang für das Innere des Endoskops (2) und/oder eines Kanalsystems eines Reinigungs- und Desinfektionsgeräts (10) und/oder einer Vorrichtung zur Dichtigkeitsüberprüfung (20) vor oder nach einem Reinigungs- und/oder Desinfektionsvorgang einzuschalten.

9. Verwendung eines Luftfeuchtigkeitssensors (24) in einer Vorrichtung (20) zur Dichtigkeitsüberprüfung eines Reinigungs- und Desinfektionsgeräts (10) für Endoskope nach Anspruch 7 oder 8 zur Feststellung einer Feuchtigkeit in einem Endoskop (2).

## Claims

1. A method for the treatment of endoscopes, wherein an endoscope (2) is attached to a compressed-air hose (23) connected to a compressed-air source (22) in an compressed air-tight manner and is supplied with a predefined or predefinable quantity or with a predefined or predefinable pressure of compressed air, configuring a first excess pressure with respect to an ambient pressure, wherein during a measuring period with an interrupted connection to the compressed-air source (22) a drop in compressed air is measured and following the end of the measuring period or after falling below a lower limit pressure, the compressed air is wholly or partially let out again and an imperviousness of the endoscope (2) is determined from a course of the drop in pressure, and the endoscope (2) is subsequently treated by applying a second excess pressure which is lower than the first excess pressure, **characterized in that** at least one first measurement of the humidity of the compressed air is taken during the inflation of the endoscope (2) or during the treatment of the endoscope (2) and at least one second measurement of the humidity of the compressed air is taken at a later time when the compressed air is let out of the endoscope (2) or during the treatment of the endoscope (2), and the humidity in the endoscope is established on the basis of a difference or trend in the humidity measurements.

2. The method according to Claim 1, **characterized in that** in the event of a difference or a trend in the humidity measurements, which is above a predetermined or predeterminable lower limit, excessive humidity in the endoscope (2) is ascertained and an indication thereof is output.

3. The method according to Claim 1 or 2, **characterized in that** in the event of excessive humidity being present in the endoscope (2) prior to or following a washing and disinfecting of the endoscope (2), an additional drying of an internal space of the endoscope (2) and/or of a channel system of a washing and disinfection device (10) and/or of a device for checking imperviousness (20) is performed.

4. The method according to any one of Claims 1 to 3, **characterized in that** in the event of considerably excessive humidity in the endoscope (2) above a second limit, which is above the predetermined or predeterminable lower limit, or in the event that excessive humidity occurs multiple times, in particular at least twice, in the endoscope (2), damage of the endoscope (2) is ascertained and an indication thereof is output.

5. A device for the treatment of endoscopes, comprising a compressed-air source (22), a compressed-air hose (23), an air pressure measuring device, a flushing unit (30) and a control device (12), wherein the compressed-air hose is or can be connected, on the one hand, to the compressed-air source (23) and, on the other hand, to an endoscope (2) to be treated, wherein the air pressure measuring device is configured to measure an air pressure of an endoscope (2) connected to the compressed-air source (22) via the compressed-air hose (23), wherein the control device (12) is configured and designed, for an imperviousness test, to supply the endoscope (2) with compressed air from the compressed-air source (22) to a first excess pressure with respect to an ambient pressure, to measure a temporally dependent drop in compressed air in the endoscope (2) supplied with compressed air during an interrupted connection to the compressed-air source (22), following the end of the measuring period or after falling below a lower limit pressure, to let out the compressed air again wholly or partially, and to determine an imperviousness of the endoscope (2) from a course of the drop in pressure, and to subsequently treat the endoscope (2) by applying a second excess pressure which is lower than the first excess pressure, **characterized in that** an humidity sensor (24) is additionally comprised, which is connected via a signal and/or data connection (26) to the control device (12) and is configured and arranged to measure the humidity in an air volume between the endoscope (2) and the compressed-air source (22), in particular in the compressed-air hose (23).

6. The device (20) according to Claim 5, **characterized in that** the control device (12) is configured and designed to perform a method according to any one of Claims 1 to 5 and to establish the humidity in the endoscope (2) from a difference or trend in humidity measurements of the compressed air when the endoscope (2) is inflated and the compressed air is let out of the endoscope (2).

7. An endoscope washing and disinfection device (10) having a device (20) for checking the imperviousness of endoscopes according to Claim 5 or 6.

8. The endoscope washing and disinfection device (10) according to Claim 7, **characterized in that** a control unit of the washing and disinfection device (10) comprises the control device (12) of the device (20) for checking the imperviousness of endoscopes and is configured and designed, after ascertaining excessive humidity in an endoscope (2), to switch on a drying process for the interior of the endoscope (2) and/or of a channel system of a washing and disinfection device (10) and/or of a device for checking the imperviousness (20) prior to or following a washing and/or disinfecting process.

9. Use of an air humidity sensor (24) in a device (20) for checking the imperviousness of an endoscope washing and disinfection device (10) according to Claim 7 or 8 in order to ascertain the humidity in an endoscope (2).

## Revendications

1. Procédé de traitement d'endoscopes, dans lequel un endoscope (2) est raccordé, de manière étanche à l'air comprimé, à un tuyau à air comprimé (23) relié à une source d'air comprimé (22) et est soumis à une quantité d'air comprimé prédéterminée ou prédéfinie ou à une pression d'air comprimé prédéterminée ou prédéfinie avec formation d'une première surpression par rapport à une pression ambiante, une chute d'air comprimé étant mesurée pendant une période de mesure lorsque la connexion à la source d'air comprimé (22) est interrompue, et après que la période de mesure a expiré ou qu'une pression limite inférieure a été dépassée, l'air comprimé étant à nouveau évacué complètement ou partiellement et une étanchéité de l'endoscope (2) étant déterminée à partir d'une évolution de la chute de pression, et l'endoscope (2) est ensuite traité en utilisant une deuxième surpression qui est inférieure à la première surpression, **caractérisé en ce qu'**au moins une première mesure d'une humidité de l'air comprimé est effectuée pendant le gonflage de l'endoscope (2) ou pendant le traitement de l'endoscope (2) et au moins une deuxième mesure d'une humidité de l'air comprimé est effectuée à un moment ultérieur lorsque l'air comprimé est évacué de l'endoscope (2) ou pendant le traitement de l'endoscope (2), et une humidité dans l'endoscope est déterminée sur la base d'une différence ou d'une tendance des mesures d'humidité.

2. Procédé selon la revendication 1, **caractérisé en ce que**, dans le cas où une différence ou une tendance des mesures d'humidité est supérieure à une valeur limite inférieure prédéterminée ou apte à être prédéterminée, une humidité excessive dans l'endoscope (2) est déterminée et une indication de celle-ci est émise.

3. Procédé selon la revendication 1 ou la revendication 2, **caractérisé en ce que**, en présence d'une humidité excessive dans l'endoscope (2), un séchage supplémentaire d'un intérieur de l'endoscope (2) et/ou d'un système de canaux d'un dispositif de nettoyage et de désinfection (10) et/ou d'un dispositif de contrôle d'étanchéité (20) est effectué avant ou après un nettoyage et une désinfection de l'endoscope (2).

4. Procédé selon l'une des revendications 1 à 3, **caractérisée en ce que**, en cas d'humidité fortement excessive dans l'endoscope (2) au-dessus d'une deuxième valeur limite qui est supérieure à la valeur limite inférieure prédéterminée ou apte à être prédéterminée, ou en cas d'apparitions répétées, en particulier au moins deux de ces apparitions, d'une humidité excessive dans l'endoscope (2), il est détecté un endommagement de l'endoscope (2) et une indication de celui-ci est émise.

5. Dispositif de traitement d'endoscopes, comprenant une source d'air comprimé (22), un tuyau à air comprimé (23), un dispositif de mesure de la pression d'air, une unité de rinçage (30) et un dispositif de commande (12), le tuyau à air comprimé étant raccordé ou apte à être raccordé, d'une part, à la source d'air comprimé (23) et, d'autre part, à un endoscope (2) à traiter, le dispositif de mesure de la pression d'air étant conçu pour mesurer une pression d'air d'un endoscope (2) relié à la source d'air comprimé (22) par le tuyau d'air comprimé (23), le dispositif de commande (12) étant conçu et agencé pour appliquer à l'endoscope (2) de l'air comprimé provenant de la source d'air comprimé (22) jusqu'à une première surpression par rapport à une pression ambiante, en vue d'un test d'étanchéité lorsque le raccordement à la source d'air comprimé (22) est interrompu, pour mesurer une chute d'air comprimé en fonction du temps au niveau de l'endoscope (2) auquel l'air comprimé est appliqué, pour, après que la période de mesure se soit écoulée ou que la pression soit tombée en dessous d'une pression limite inférieure, évacuer à nouveau tout ou partie de l'air comprimé et déterminer une étanchéité de l'endoscope (2) à partir d'une progression de la chute de pression, puis de traiter l'endoscope (2) en utilisant une deuxième surpression qui est inférieure à la première surpression, **caractérisé en ce qu'**un capteur d'humidité (24) est en outre inclus, qui est relié au dispositif de commande (12) via une connexion (26) de signaux et/ou de données et est conçu et agencé pour mesurer une humidité dans un volume d'air entre l'endoscope (2) et la source d'air comprimé (22), en particulier dans le tuyau d'air comprimé (23).

6. Dispositif (20) selon la revendication 5, **caractérisé en ce que** le dispositif de commande (12) est conçu et agencé pour mettre en œuvre un procédé selon l'une quelconque des revendications 1 à 5 et pour déterminer une humidité dans l'endoscope (2) à partir d'une différence ou d'une tendance dans les mesures d'humidité de l'air comprimé lorsque l'endoscope (2) est gonflé et lorsque l'air comprimé est évacué de l'endoscope (2).

7. Appareil (10) de nettoyage et de désinfection pour endoscopes comprenant un dispositif (20) pour le contrôle de l'étanchéité d'endoscopes selon la revendication 5 ou la revendication 6.

8. Appareil (10) de nettoyage et de désinfection pour endoscopes selon la revendication 7, **caractérisé en ce qu'**une unité de commande de l'appareil (10) de nettoyage et de désinfection comprend le dispositif de commande (12) du dispositif (20) pour le contrôle de l'étanchéité des endoscopes, et est conçue et agencée pour, après détection d'une humidité excessive dans un endoscope (2), mettre en œuvre une opération de séchage pour l'intérieur de l'endoscope (2) et/ou d'un système de canaux d'un appareil de nettoyage et de désinfection (10) et/ou d'un dispositif de contrôle d'étanchéité (20) avant ou après une opération de nettoyage et/ou de désinfection.

9. Utilisation d'un capteur d'humidité de l'air (24) dans un dispositif de contrôle d'étanchéité (20) d'un appareil de nettoyage et de désinfection d'endoscopes (10) selon la revendication 7 ou la revendication 8 pour détecter une humidité dans un endoscope (2).
